# EUROPÄISCHE PATENTANMELDUNG

(11) **EP 3 646 918 A1**
(43) Veröffentlichungstag der Anmeldung: **06.05.2020**
(21) Anmeldenummer: 18204241.6
(22) Anmeldetag: 05.11.2018
(51) Int. Cl.: A61M 37/00, B25D 17/24

(54) **VORRICHTUNG ZUM AUFSTECHEN EINES ORGANISCHES GEWEBES SOWIE ANTRIEBSMODUL**

(71) Anmelder: MT.DERM, 14167 Berlin (DE)
(72) Erfinder: Scherkowski, Dirk, 12489 Berlin (DE); Röthig, Sebastian, 10553 Berlin (DE)
(74) Vertreter: Bittner, Thomas L.

(57) **Zusammenfassung**

Die Offenbarung betrifft eine Vorrichtung zum Aufstechen eines organischen Gewebes, insbesondere einer menschlichen oder tierischen Haut. Die Vorrichtung umfasst ein Gehäuse (1), eine Stecheinrichtung (2), die in dem Gehäuse (1) an einem distalen Ende aufgenommen ist, und eine Antriebseinrichtung, die in dem Gehäuse gebildet und proximal der Stecheinrichtung (2) angeordnet ist. Die Antriebseinrichtung umfasst einen Antrieb (4), der eingerichtet ist, im Betrieb eine kontinuierliche Antriebsrotation bereitzustellen, ein erstes Antriebselement (6; 20), ein zweites Antriebselement (8) und ein Gegenelement (11; 18). Weiterhin ist ein Antriebsmodul für eine Vorrichtung zum Aufstechen eines organischen Gewebes vorgesehen.

## Beschreibung

Die Erfindung betrifft eine Vorrichtung zum Aufstechen eines organischen Gewebes sowie ein Antriebsmodul.

### Hintergrund

Derartige Vorrichtungen werden benutzt, um organisches Gewebe, insbesondere menschliche oder tierische Haut, mit einer oder mehreren Gewebeöffnungen zu versehen. Anschließend kann durch die Gewebeöffnungen gegebenenfalls ein fluider Wirkstoff in das Gewebe eingebracht werden. Beispielsweise dienen derartige Stechvorrichtungen zum Eintrag eines farbigen Stoffs in Verbindung mit dem Tätowieren oder dem Ausbilden von permanentem Make-up. Aber auch das Einbringen anderer Wirkstoffe, wie medizinischer oder kosmetischer Substanzen, kann in diesem Zusammenhang vorgesehen sein.

Bei bekannten Stechvorrichtungen wird üblicherweise eine Stecheinrichtung, die mit einer oder mehreren Nadeln versehen ist, an einem Gehäuse gehalten und mit Hilfe einer Antriebseinrichtung im Betrieb oszillierend vor und zurück bewegt, so dass bei entsprechender räumlicher Beziehung zueinander ein wiederholtes Aufstechen des organischen Gewebes stattfindet. Die oszillierende Bewegung von Elementen der Antriebseinrichtung, und schließlich der Stecheinrichtung, führt dazu, dass die als Handgerät ausgebildete Vorrichtung vibriert. Diese Schwingungen muss der Nutzer dann mit seiner Hand, in welcher er die Stechvorrichtung hält, auffangen. Das Vibrieren der Vorrichtung wird vom Nutzer als unangenehm empfunden.

Das Dokument EP 1 495 782 A1 betrifft eine Vorrichtung zum Aufbringen einer Tätowierung oder von permanentem Make-Up. Es werden verschiedene Ausgestaltungen eines Antriebsmechanismus der Vorrichtung zum Aufbringen einer Tätowierung oder von permanentem Make-Up beschrieben.

In dem Dokument EP 2 594 312 A1 ist eine Vorrichtung zum Aufstechen eines organischen Gewebes offenbart, bei der ein Gegenbauteil bei einer oszillierenden Antriebsbewegung eines Antriebsbauteils oszillierend und gegenläufig zum Antriebsbauteil bewegt wird.

### Zusammenfassung

Aufgabe der Erfindung ist es, eine Vorrichtung zum Aufstechen eines organischen Gewebes sowie ein Antriebsmodul für eine solche Vorrichtung anzugeben, die über einen verbesserten Nutzerkomfort verfügen, insbesondere dadurch, dass Schwingungen der Vorrichtung oder von Elementen hiervon gemindert oder ganz unterbunden sind.

Zur Lösung der Aufgabe sind eine Vorrichtung zum Aufstechen eines organischen Gewebes gemäß dem unabhängigen Anspruch 1 sowie ein Antriebsmodul für eine Vorrichtung zum Aufstechen eines organischen Gewebes gemäß dem unabhängigen Anspruch 12 geschaffen. Ausgestaltungen sind Gegenstand von abhängigen Unteransprüchen.

Nach einem Aspekt ist eine Vorrichtung zum Aufstechen eines organischen Gewebes, insbesondere einer menschlichen oder tierischen Haut, geschaffen. Die Vorrichtung umfasst ein Gehäuse, eine Stecheinrichtung, die in dem Gehäuse an einem distalen Ende aufgenommen ist, und eine Antriebseinrichtung, die in dem Gehäuse gebildet und proximal der Stecheinrichtung angeordnet ist. Die Antriebseinrichtung umfasst einen Antrieb, der eingerichtet ist, im Betrieb eine kontinuierliche Antriebsrotation bereitzustellen, ein erstes Antriebselement, ein zweites Antriebselement und ein Gegenelement. Das erste Antriebselement ist distal des Antriebs angeordnet und koppelt funktionell an den Antrieb, derart, dass die kontinuierliche Antriebsrotation des Antriebs auf das erste Antriebselement eingekoppelt wird und zumindest ein Abschnitt des ersten Antriebselements im Betrieb eine kontinuierliche Rotationsbewegung ausführt. Das zweite Antriebselement ist im Wesentlichen distal des ersten Antriebselements angeordnet und koppelt funktionell über einen ersten Kopplungspunkt an das erste Antriebselement. Die kontinuierliche Rotationsbewegung des zumindest einen Abschnitts des ersten Antriebselements bewirkt im Betrieb eine oszillierende Bewegung des ersten Kopplungspunkts zwischen einer proximalen Position und einer distalen Position des ersten Kopplungspunkts. Die oszillierende Bewegung des ersten Kopplungspunkts wird auf das zweite Antriebselement eingekoppelt, derart, dass das zweite Antriebselement eine oszillierende Antriebsbewegung ausführt. Das zweite Antriebselement koppelt funktionell an die Stecheinrichtung, derart, dass die oszillierende Antriebsbewegung des zweiten Antriebselements auf die Stecheinrichtung eingekoppelt wird. Das Gegenelement ist distal des Antriebs angeordnet und koppelt funktionell über einen zweiten Kopplungspunkt an das erste Antriebselement. Die kontinuierliche Rotationsbewegung des zumindest einen Abschnitts des ersten Antriebselements bewirkt im Betrieb eine oszillierende Bewegung des zweiten Kopplungspunkts zwischen einer proximalen Position und einer distalen Position des zweiten Kopplungspunkts. Die oszillierende Bewegung des zweiten Kopplungspunkts wird auf das Gegenelement eingekoppelt, derart, dass das Gegenelement eine oszillierende Elementbewegung ausführt. Die oszillierende Bewegung des ersten Kopplungspunkts und die oszillierende Antriebsbewegung des zweiten Antriebselements sind gegenläufig zu der oszillierenden Bewegung des zweiten Kopplungspunkts und der oszillierenden Elementbewegung des Gegenelements, so dass in dem Gehäuse durch die oszillierende Antriebsbewegung des zweiten Antriebselements verursachte Schwingungen wenigstens teilweise durch die oszillierende Elementbewegung des Gegenelements kompensiert werden.

Nach einem weiteren Aspekt ist ein Antriebsmodul für eine Vorrichtung zum Aufstechen eines organischen Gewebes, insbesondere einer menschlichen oder tierischen Haut, geschaffen. Das Antriebsmodul weist ein Modulgehäuse und eine Antriebseinrichtung auf, die in dem Modulgehäuse gebildet und konfiguriert ist, an einem distalen Ende des Modulgehäuses an eine Stecheinrichtung eines Nadelmoduls zu koppeln. Die Antriebseinrichtung umfasst einen Antrieb, der eingerichtet ist, im Betrieb eine kontinuierliche Antriebsrotation bereitzustellen, ein erstes Antriebselement, ein zweites Antriebselement und ein Gegenelement. Das erste Antriebselement ist distal des Antriebs angeordnet und koppelt funktionell an den Antrieb, derart, dass die kontinuierliche Antriebsrotation des Antriebs auf das erste Antriebselement eingekoppelt wird und zumindest ein Abschnitt des ersten Antriebselements im Betrieb eine kontinuierliche Rotationsbewegung ausführt. Das zweite Antriebselement ist im Wesentlichen distal des ersten Antriebselements angeordnet und koppelt funktionell über einen ersten Kopplungspunkt an das erste Antriebselement, wobei die kontinuierliche Rotationsbewegung des zumindest einen Abschnitts des ersten Antriebselements im Betrieb eine oszillierende Bewegung des ersten Kopplungspunkts zwischen einer proximalen Position und einer distalen Position des ersten Kopplungspunkts bewirkt, die oszillierende Bewegung des ersten Kopplungspunkts auf das zweite Antriebselement eingekoppelt wird, derart, dass das zweite Antriebselement eine oszillierende Antriebsbewegung ausführt, und das zweite Antriebselement eingerichtet ist, funktionell an die Stecheinrichtung zu koppeln, derart, dass die oszillierende Antriebsbewegung des zweiten Antriebselements auf die Stecheinrichtung eingekoppelt wird. Das Gegenelement ist distal des Antriebs angeordnet und koppelt funktionell über einen zweiten Kopplungspunkt an das erste Antriebselement, wobei die kontinuierliche Rotationsbewegung des zumindest einen Abschnitts des ersten Antriebselements im Betrieb eine oszillierende Bewegung des zweiten Kopplungspunkts zwischen einer proximalen Position und einer distalen Position des zweiten Kopplungspunkts bewirkt, und die oszillierende Bewegung des zweiten Kopplungspunkts auf das Gegenelement eingekoppelt wird, derart, dass das Gegenelement eine oszillierende Elementbewegung ausführt. Die oszillierende Bewegung des ersten Kopplungspunkts und die oszillierende Antriebsbewegung des zweiten Antriebselements sind gegenläufig zu der oszillierenden Bewegung des zweiten Kopplungspunkts und der oszillierenden Elementbewegung des Gegenelements, so dass in dem Modulgehäuse durch die oszillierende Antriebsbewegung des zweiten Antriebselements verursachte Schwingungen wenigstens teilweise durch die oszillierende Elementbewegung des Gegenelements kompensiert werden.

In der Antriebseinrichtung verursacht der Antrieb sowohl die oszillierende Antriebsbewegung des zweiten Antriebselements als auch die oszillierende Elementbewegung des Gegenelements.

Bei der Anordnung des zweiten Antriebselements im Wesentlichen distal des ersten Antriebselements kann ein Hauptkörper des zweiten Antriebselements distal des ersten Antriebselements angeordnet sein und ein Verbindungskörper des zweiten Antriebselements kann sich zur Kopplung an dem ersten Kopplungspunkt von dem Hauptkörper zu dem ersten Kopplungspunkt erstrecken, so dass der Verbindungskörper wenigstens teilweise neben dem ersten Antriebselements angeordnet ist.

Das Gehäuse der Vorrichtung kann ein- oder mehrteilig ausgeführt sein. Im Fall einer mehrteiligen Ausführung kann vorgesehen sein, dass die Vorrichtung mit mehreren voneinander trennbaren Gehäuseteilen gebildet ist, die jeweils mit hierin aufgenommenen funktionellen Komponenten ein oder mehrere funktionelle Module bilden können, insbesondere ein Antriebsmodul mit der Antriebseinrichtung und ein hieran lösbar koppelbares Stech- oder Nadelmodul, welches die Stecheinrichtung aufnimmt. Die Module sind vorzugsweise lösbar miteinander verbunden, zum Beispiel mittels einer Steck- und / oder einer Schraubverbindung. In einer Ausgestaltung kann das Stech- oder Nadelmodul als ein Einwegmodul ausgeführt sein, welches zum Beispiel als Verbrauchsartikel in Form eines sterilisierten Moduls für die einmalige Verwendung zur Verfügung gestellt werden kann. Auch können alternativ oder ergänzend andere Funktionsmodule ans Gehäuse angekoppelt werden, zum Beispiel ein Vorrat oder Tank mit einem fluiden Stoff, welcher zum Eintrag in die Haut bestimmt sein kann und / oder zur Verbesserung der Anwendung dienen kann, beispielsweise durch Verbesserung von Gleiteigenschaften.

Die Stecheinrichtung kann mit einer Einzelnadel, einer Nadelgruppe und / oder einer Nadelplatte gebildet sein, bei der auf einer nach außen gerichteten Plattenoberfläche mehrere Nadelspitzen angeordnet sind, beispielsweise auch in Form von Mikronadelspitzen. Die Kopplung der Stecheinrichtung an das Antriebsbauteil erfolgt direkt oder vermittelt über ein oder mehrere Koppiungselemente. Alternativ oder zusätzlich zu einer oder mehreren Nadeln kann die Stecheinrichtung mit einer oder mehreren Lanzetten gebildet sein.

Eine Rotationsachse des zumindest einen Abschnitts des ersten Antriebselements kann im Wesentlichen parallel zu einer Längsachse des Gehäuses verlaufen. Es kann eine gerade Antriebseinrichtung bereitgestellt sein, die im Wesentlichen entlang einer Längsachse des Gehäuses verläuft. Alternativ kann die Rotationsachse des zumindest einen Abschnitts des ersten Antriebselements unter einem Winkel zu der Längsachse des Gehäuses verlaufen. Es kann eine gewinkelte Antriebseinrichtung bereitgestellt sein.

Das erste Antriebselement kann eine Taumelscheibe umfassen. Zum Beispiel kann auf eine Antriebswelle des Antriebs eine Taumelscheibe montiert sein, die beim Drehen der Antriebswelle eine taumelnde Bewegung ausführt. Die Taumelscheibe kann so montiert sein, dass ein proximales Ende des zweiten Antriebselements auf einer Oberfläche der Taumelscheibe aufliegt. Bei einer Drehbewegung der Antriebswelle kann dann das proximale Ende des zweiten Antriebselements durch die Taumelbewegung der Taumelscheibe vor und zurück bewegt werden. Das proximale Ende des zweiten Antriebselements kann mit Hilfe geeigneter Rückhaltemittel gegen die Oberfläche der Taumelscheibe gedrückt werden. Hierzu kann beispielsweise eine mechanische Feder genutzt werden, die zweckmäßig gegen das zweite Antriebselement drückt.

Alternativ oder zusätzlich kann auf der Antriebswelle ein Kugellager mit einem relativ zu der Antriebswelle schräg auf der Antriebswelle montierten Innenring und einem freilaufenden Außenring montiert sein, wobei der Außenring an einer Drehung gehindert wird. Beim Drehen der Antriebswelle kann das Kugellager dann eine taumelnde Bewegung ausführen und der erste und der zweite Kopplungspunkt können an dem Außenring angeordnet sein, so dass die taumelnde Bewegung des Außenrings eine jeweilige oszillierende Bewegung des ersten und des zweiten Kopplungspunkts zwischen einer jeweiligen proximalen Position und einer jeweiligen distalen Position des ersten und des zweiten Kopplungspunkts bewirkt.

Das erste Antriebselement kann eine Kurvenscheibe umfassen. Kurvenscheiben sind als solches bekannt. Das erste Antriebselement kann eine Kurvenscheibe umfassen, welche entlang des Umfangs der Kurvenscheibe ein in axialer Richtung einer Rotationsachse der Kurvenscheibe veränderliches Kurvenprofil aufweist. Das Kurvenprofil kann eine Oberfläche eines axialen Endes der Kurvenscheibe bilden. Alternativ oder zusätzlich kann das Kurvenprofil auf einer Mantelfläche der Kurvenscheibe angeordnet sein, beispielsweise als eine Nut in der Mantelfläche und/oder als eine Erhebung auf der Mantelfläche.

Das zweite Antriebselement kann lose mit dem ersten Antriebselement gekoppelt sein. Zum Beispiel kann das zweite Antriebselement an dem ersten Kopplungspunkt an dem ersten Antriebselement anliegen. Für das Einkoppeln der oszillierenden Bewegung des ersten Kopplungspunkts auf das zweite Antriebselement kann eine Antriebskraft nur in eine erste axiale Richtung über den ersten Kopplungspunkt auf das zweite Antriebselement aufgebracht werden. Das zweite Antriebselement kann mit einem Rückstellelement, beispielsweise mit einem Federelement, gekoppelt sein, welches eine Rückstellkraft auf das zweite Antriebselement ausübt, um das zweite Antriebselement in eine der ersten axialen Richtung entgegengesetzte zweite axiale Richtung zurückzustellen.

Alternativ kann das zweite Antriebselement, zumindest bezogen auf einen Freiheitsgrad, starr mit dem ersten Antriebselement gekoppelt sein. Hierdurch kann für das Einkoppeln der oszillierenden Bewegung des ersten Kopplungspunkts auf das zweite Antriebselement eine Antriebskraft sowohl in eine axiale Richtung als auch entgegen der axialen Richtung über den ersten Kopplungspunkt auf das zweite Antriebselement aufgebracht werden. Die Kopplung zwischen dem ersten Antriebselement und dem zweiten Antriebselement kann, bezogen auf einen Freiheitsgrad, starr und, bezogen auf einen weiteren Freiheitsgrad, beweglich sein. Zum Beispiel kann die Kopplung, bezogen auf eine Translation, in wenigstens einer Richtung starr und, bezogen auf eine Rotation, um wenigstens eine Achse beweglich sein.

Das Gegenelement kann seitlich des ersten Antriebselements angeordnet und starr mit dem ersten Antriebselement verbunden sein. Zum Beispiel kann das Gegenelement mittels Schrauben, Schweißen, Löten und/oder Kleben seitlich an dem ersten Antriebselement fixiert sein.

Die oszillierende Elementbewegung des Gegenelements kann eine im Wesentlichen kreisbogenförmige oszillierende Bewegung sein. In einem Beispiel umfasst das erste Antriebselement eine Taumelscheibe, derart, dass die taumelnde Bewegung der Taumelscheibe eine kreisbogenförmige oszillierende Bewegung des zweiten Kopplungspunkts bewirkt, und das Gegenelement ist starr mit dem ersten Antriebselement verbunden, so dass die kreisbogenförmige oszillierende Bewegung des zweiten Kopplungspunkts eine kreisbogenförmige oszillierende Bewegung des Gegenelements bewirkt.

Das Gegenelement kann im Wesentlichen distal des ersten Antriebselements angeordnet und im Wesentlichen wie das zweite Antriebselement geformt sein. Hierbei können die in Verbindung mit dem zweiten Antriebselement erläuterten Ausgestaltungen entsprechend vorgesehen sein, insbesondere bezüglich der Kopplung zwischen dem ersten Antriebselement und dem Gegenelement.

Eine Masse des Gegenelements kann im Wesentlichen gleich einer Masse des zweiten Antriebselements sein. Das zweite Antriebselement und das Gegenelement können bei der jeweiligen oszillierenden Bewegung mit gleicher Schwingungsamplitude verlagert werden. Alternativ kann vorgesehen sein, dass die Masse des Gegenelements größer als die Masse des ersten Antriebselements ist, zum Beispiel zum wenigstens teilweisen Ausgleich der Masse der Stecheinrichtung, die an das zweite Antriebselement koppelt, und / oder anderer, an das zweite Antriebselement koppelnder Bauteile, die bei der oszillierenden Bewegung des zweiten Antriebselements mitbewegt werden. Hierbei kann dann vorgesehen sein, dass das Antriebselement und das Gegenelement eine gegenläufige Bewegung mit unterschiedlicher Schwingungsamplitude ausführen.

Für den, zumindest teilweisen, Ausgleich der in dem Gehäuse durch die oszillierende Antriebsbewegung des zweiten Antriebselements verursachten Schwingungen durch die oszillierende Elementbewegung des Gegenelements können eine Masse des Gegenelements und ein Abstand des Massenschwerpunkts des Gegenelements von der Längsachse des Gehäuses auf eine Masse des zweiten Antriebselements und einen Abstand des Massenschwerpunkts des zweiten Antriebselements von der Längsachse des Gehäuses abgestimmt sein. Zusätzlich können die Masse und die Lage des Massenschwerpunkts eines oder mehrerer weiterer Elemente bei der Gestaltung des Gegenelements Berücksichtigung finden. Bei den weiteren Elementen kann es sich beispielweise um die Stecheinrichtung und / oder um andere, an das zweite Antriebselement koppelnde Bauteile handeln, die bei der oszillierenden Bewegung des zweiten Antriebselements mitbewegt werden.

In einem Beispiel ist die Masse des Gegenelements im Wesentlichen gleich der Gesamtmasse der Elemente, deren Masse auszugleichen ist, und ein Abstand des Massenmittelpunkts des Gegenelements von der Längsachse des Gehäuses ist im Wesentlichen gleich einem Abstand des gemeinsamen Massenmittelpunkts der Elemente. deren Masse auszugleichen ist, von der Längsachse des Gehäuses.

Die Bewegungsbahnen des zweiten Antriebselements und des Gegenelements können im Wesentlichen parallel sein. Ein Abstand zwischen den jeweiligen Bewegungsbahnen des zweiten Antriebselements und des Gegenelements kann möglichst klein sein. Insbesondere kann ein Abstand zwischen dem Massenmittelpunkt des Gegenelements und dem gemeinsamen Massenmittelpunkt der Elemente, deren Masse auszugleichen ist, minimiert sein.

Der Antrieb kann ein Elektromotor sein. Insbesondere kann der Antrieb ein Gleichstrommotor sein, zum Beispiel ein elektronisch kommutierter bzw. bürstenloser Gleichstrommotor.

In dem Gehäuse können, zumindest entlang einer Bewegungsrichtung der Stecheinrichtung, durch die oszillierende Antriebsbewegung des zweiten Antriebselements verursachte Schwingungen im Wesentlichen vollständig durch die oszillierende Elementbewegung des Gegenelements kompensiert werden. Hierzu können die Masse und die Anordnung des Gegenelements auf das zweite Antriebselement abgestimmt sein, derart, dass im Betrieb der Betrag eines Impulses des Gegenelements entlang der Bewegungsrichtung gleich dem Betrag eines Impulses des zweiten Antriebselements entlang der Bewegungsrichtung und diesem entgegengesetzt ist. Hierbei können Impulsanteile in anderen Richtungen als der Bewegungsrichtung der Stecheinrichtung unberücksichtigt bleiben. Zusätzlich zum Impuls des zweiten Antriebselements können der Impuls der Stecheinrichtung, die an das zweite Antriebselement koppelt, und / oder anderer an das zweite Antriebselement koppelnder Bauteile, die bei der oszillierenden Bewegung des zweiten Antriebselements mitbewegt werden, berücksichtigt werden.

Bei einer Kurvenscheibe kann die Anordnung des ersten Kopplungspunkts an dem ersten Antriebselement veränderlich und die Anordnung des ersten Kopplungspunkts an dem zweiten Antriebselements kann unveränderlich sein. Die Anordnung des zweiten Kopplungspunkts an dem ersten Antriebselement kann veränderlich und die Anordnung des zweiten Kopplungspunkts an dem Gegenelement kann unveränderlich sein.

Die in Verbindung mit der Vorrichtung zum Aufstechen eines organischen Gewebes erläuterten Ausgestaltungen können bei dem Antriebsmodul für eine Vorrichtung zum Aufstechen eines organischen Gewebes entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine Teilschnitt-Ansicht einer Vorrichtung zum Aufstechen eines organischen Gewebes,
- Fig. 2: eine Schnittansicht einer anderen Vorrichtung zum Aufstechen eines organischen Gewebes,
- Fig. 3a: eine Teilschnitt-Ansicht einer weiteren Vorrichtung zum Aufstechen eines organischen Gewebes,
- Fig. 3b: eine Teilschnitt-Ansicht der Vorrichtung aus der Fig. 3a in einer anderen Stellung.
- Fig. 4a: eine schematische Darstellung einer Antriebseinrichtung für eine Vorrichtung zum Aufstechen eines organischen Gewebes und
- Fig. 4b: eine schematische Darstellung der Antriebseinrichtung aus der Fig. 4a in einer anderen Stellung,

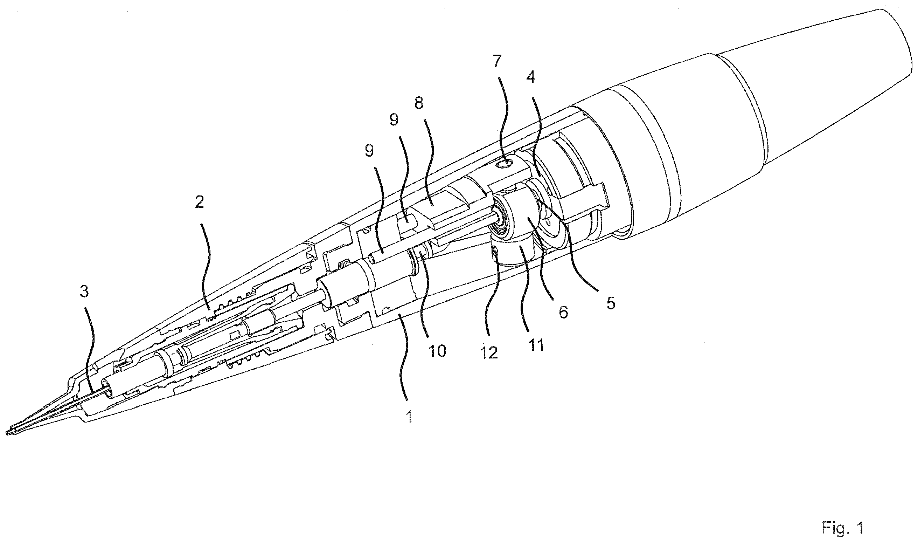

Die Fig. 1 zeigt eine Vorrichtung zum Aufstechen eines organischen Gewebes, insbesondere einer menschlichen oder tierischen Haut. An einem distalen Ende eines Gehäuses 1 ist eine Stecheinrichtung 2 aufgenommen. In der gezeigten Ausführung ist die Stecheinrichtung 2 mit mehreren Nadeln 3 gebildet. Alternativ kann die Stecheinrichtung 2 mit nur einer Nadel oder mit einer oder mehreren Lanzetten gebildet sein. Im Betrieb führen die mehreren Nadeln 3 eine oszillierende Linearbewegung aus, um das organische Gewebe aufzustechen. Hierzu ist in dem Gehäuse 1 ein Elektromotor 4 als Antrieb angeordnet. Auf einer Motorwelle 5 des Motors 4 ist ein erstes Antriebselement angeordnet. In der Ausführung gemäß der Fig. 1 handelt es sich bei dem ersten Antriebselement um ein Taumelscheiben-Element 6.

Eine kontinuierliche Rotation der Motorwelle 5 führt zu einer Taumelbewegung des Taumelscheiben-Elements 6, welche eine oszillierende kreisbogenförmige Bewegung eines ersten Kopplungspunkts, welcher als Zapfen 7 bereitgestellt ist, um einen Mittelpunkt des Taumelschreiben-Elements 6 zwischen einer proximalen Position und einer distalen Position bewirkt. Der Zapfen 7 ist in einer Bohrung eines zweiten Antriebselements 8 angeordnet. Das zweite Antriebselement 8 ist auf Führungsachsen 9 linear gelagert, so dass das zweite Antriebselement 8 eine lineare Antriebsbewegung zwischen einer proximalen und einer distalen Stellung des zweiten Antriebselements 8 ausführen kann. Die Antriebsbewegung des zweiten Antriebselements 8 wird über einen Stößel 10 auf die Stecheinrichtung 2 eingekoppelt, derart, dass die Nadeln 3 im Betrieb eine lineare Stechbewegung ausführen.

Durch die Anordnung des Zapfens 7 in der Bohrung des zweiten Antriebselements 8 wird die oszillierende Bewegung des Zapfens 7 auf das zweite Antriebselement 8 übertragen. Die resultierende oszillierende Antriebsbewegung des zweiten Antriebselements 8 führt dann zu einer oszillierenden linearen Stechbewegung der Nadeln 3. Gleichzeitig wird durch die Linearführung des zweiten Antriebselements 8 und die Anordnung des Zapfens 7 in der Bohrung des zweiten Antriebselements 8 eine Rotation des Zapfens 7 um die Motorwelle 5 verhindert.

Auf einer, dem ersten Kopplungspunkt gegenüberliegenden, Seite des ersten Antriebselements 6 ist auf einem als zweiter Kopplungspunkt dienenden zweiten Zapfen (nicht dargestellt) ein Gegenelement angeordnet. Das Gegenelement ist als eine Ausgleichsmasse 11, z.B. aus Messing, bereitgestellt, welche mittels einer Madenschraube 12 auf dem zweiten Zapfen befestigt ist. Bei einer kontinuierlichen Rotation der Motorwelle 5 führt die Taumelbewegung des Taumelscheiben-Elements 6 zu einer, der Bewegung des ersten Kopplungspunkts gegenläufigen, oszillierenden kreisbogenförmigen Bewegung des zweiten Kopplungspunkts und der Ausgleichsmasse 11 um den Mittelpunkt des Taumelschreiben-Elements 6 zwischen einer proximalen Position und einer distalen Position. Der Motor 4 treibt somit sowohl die oszillierende Bewegung des zweiten Antriebselements 8 als auch die oszillierende Bewegung der Ausgleichsmasse 11 an.

Der zu der Rotationsachse der Motorwelle 5 parallele Linearanteil der oszillierenden Bewegung der Ausgleichsmasse 11 wirkt dem zu der Rotationsachse der Motorwelle 5 parallelen Linearanteil einer durch die oszillierenden Bewegung des zweiten Antriebselements 8 verursachten Vibration entgegen und gleicht diese, zumindest teilweise, aus.

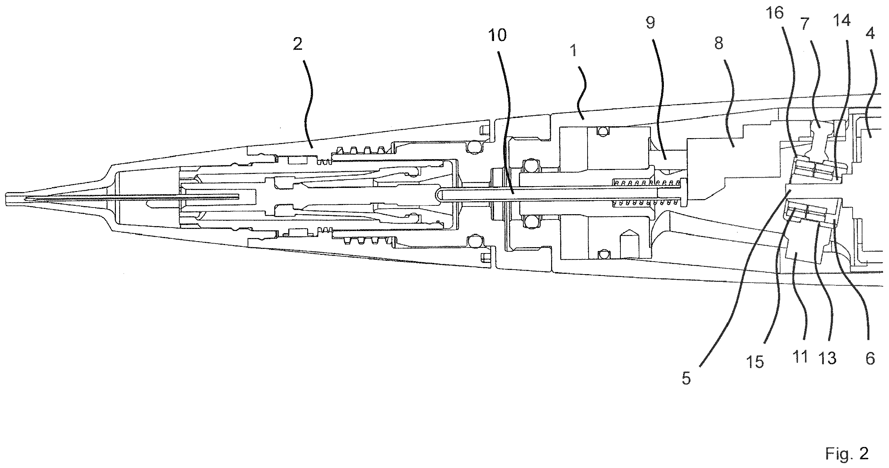

Die Fig. 2 zeigt eine andere Vorrichtung zum Aufstechen eines organischen Gewebes. Im Vergleich zu der Vorrichtung gemäß der Fig. 1 ist in der Vorrichtung der Fig. 2 die Ausgleichsmasse aus Stahl gefertigt. Der zweite Kopplungspunkt ist als eine Öffnung 13 des Taumelscheiben-Elements 6 bereitgestellt, in welcher die Ausgleichsmasse 11 angeordnet ist. Die Ausgleichsmasse 11 ist an das Taumelscheiben-Element 6 angeschweißt.

In der Fig. 2 ist die Funktionsweise eines beispielhaften Taumelscheiben-Elements 6 erkennbar: Ein inneres Element 14 des Taumeischeiben-Elements 6 ist schräg auf der Motorwelle 5 angeordnet, so dass eine Rotation der Motorwelle 5 zu einer Rotation des inneren Elements 14 um die Rotationsachse der Motorwelle 5 führt. Hierzu ist im vorliegenden Beispiel ein rotationssymmetrischer Grundkörper des inneren Elements 14 mit einer Bohrung versehen, welche schräg, also nicht parallel oder senkrecht, zu der Symmetrieachse des rotationssymmetrischen Grundkörpers verläuft. Die Motorwelle 5 ist in der Bohrung angeordnet, so dass die Symmetrieachse des rotationssymmetrischen Grundkörpers des inneren Elements 14 schräg zu der Motorwelle verläuft. Eine Rotation der Motorwelle 5 führt somit zu einer taumelnden Bewegung des inneren Elements 14. Das innere Element 14 ist über zwei Wälzlager 15 mit einem äußeren Element 16 des Taumelscheiben-Elements 6 verbunden. Die Öffnung 13 ist in dem äußeren Element 16 angeordnet. Weiterhin ist der Zapfen 7 an dem äußeren Element 16 angeordnet und mit diesem verbunden. Durch die Anordnung des Zapfens 7 in der Bohrung des zweiten Antriebselements 8 ist eine Rotation des äußeren Elements 16 verhindert. Hierdurch führt bei einer Rotation der Motorwelle 6 die rotierende Taumelbewegung des inneren Elements 14 zu einer Kippbewegung des äußeren Elements 16 und einer oszillierenden kreisbogenförmigen Bewegung des Zapfens 7. Die oszillierende kreisbogenförmige Bewegung des Zapfens 7 bewirkt eine oszillierende lineare Antriebsbewegung des zweiten Antriebselements 8 entlang der Führungsachsen 9, welche auf die Stecheinrichtung 2 eingekoppelt wird.

Für den zumindest teilweisen Ausgleich des zu der Rotationsachse der Motorwelle 5 parallelen Linearanteils der durch die oszillierende Bewegung des zweiten Antriebselements 8 verursachten Vibration durch die oszillierende Bewegung der Ausgleichsmasse 11 können die Masse der Ausgleichsmasse 11 und ein Abstand des Massenschwerpunkts der Ausgleichsmasse 11 von der Längsachse des Gehäuses 1 auf eine Masse des zweiten Antriebselements 8 und einen Abstand des Massenschwerpunkts des zweiten Antriebselements 8 von der Längsachse des Gehäuses 1 abgestimmt sein. Zusätzlich können die Masse und die Lage des Massenschwerpunkts eines oder mehrerer weiterer Elemente bei der Gestaltung der Ausgleichsmasse 11 Berücksichtigung finden. Bei den weiteren Elementen kann es sich beispielweise um die Stecheinrichtung 2 und / oder andere, an das zweite Antriebselement 8 koppelnde, Bauteile handeln, die bei der oszillierenden Bewegung des zweiten Antriebselements 8 mitbewegt werden.

Gemäß einem Beispiel ist die Gesamtmasse des Zapfens 7, des zweiten Antriebselements 8 und des Stößels 10 gleich der Masse der Ausgleichsmasse 11 und der Abstand des kombinierten Massenschwerpunkts des Zapfens 7, des zweiten Antriebselements 8 und des Stößels 10 von der Längsachse des Gehäuses 1 ist gleich dem Abstand des Massenschwerpunkts der Ausgleichsmasse 11 von der Längsachse des Gehäuses 1. Der jeweilige Abstand der Massenschwerpunkte von der Längsachse des Gehäuses 1 ist hierbei für eine Ausrichtung der Komponenten angegeben, in welcher der erste und der zweite Kopplungspunkt in einer Längsrichtung des Gehäuses 1 mittig zwischen der proximalen Position und der distalen Position des jeweiligen Kopplungspunkts angeordnet sind.

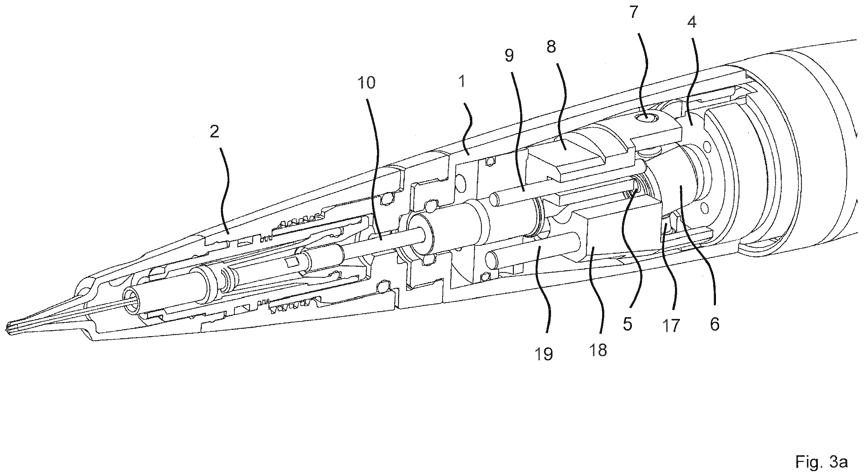

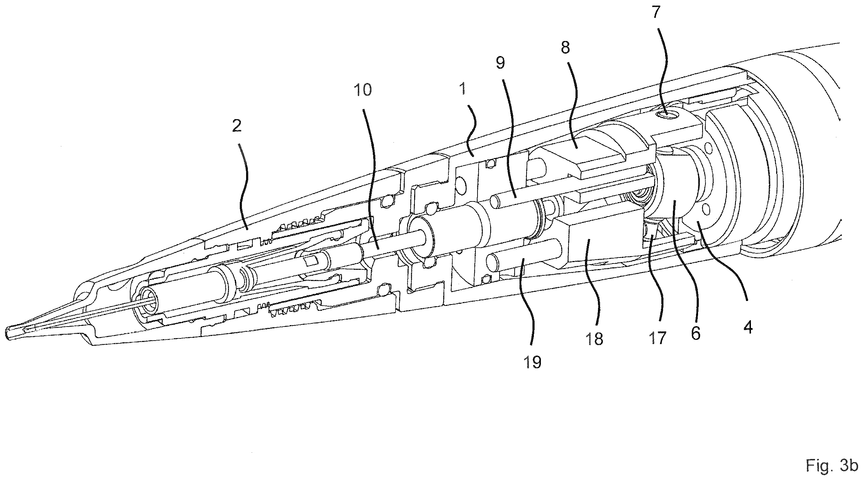

Die Figuren 3a und 3b zeigen eine weitere Vorrichtung zum Aufstechen eines organischen Gewebes in unterschiedlichen Stellungen der Motorwelle 5. Gemäß den Figuren 3a und 3b ist der zweite Kopplungspunkt mit einem weiteren Zapfen 17 gebildet, der durch eine kontinuierliche Rotation der Motorwelle 5 und die Taumelbewegung des Taumelscheiben-Elements 6 in eine oszillierende kreisbogenförmige Bewegung um den Mittelpunkt des Taumelschreiben-Elements 6 zwischen einer proximalen Position und einer distalen Position versetzt wird. Der weitere Zapfen 17 ist in einer Aussparung eines als Schiebeelement 18 ausgebildeten Gegenelements angeordnet. Das Schiebeelement 18 ist auf Führungsachsen 19 linear gelagert, so dass das Schiebeelement 18 eine lineare Bewegung zwischen einer proximalen und einer distalen Stellung des Schiebeelements 18 ausführen kann. Durch die Anordnung des weiteren Zapfens 17 in der Aussparung des Schiebeelements 18 wird die oszillierende Bewegung des weiteren Zapfens 17 auf das Schiebeelement 18 übertragen. Die resultierende oszillierende lineare Bewegung des Schiebeelements 18 ist der oszillierenden Antriebsbewegung des zweiten Antriebselements 8 gegenläufig.

Die oszillierende lineare Bewegung des Schiebeelements 18 wirkt dem zu der Rotationsachse der Motorwelle 5 parallelen Linearanteil einer durch die oszillierende Bewegung des zweiten Antriebselements 8 verursachten Vibration entgegen und gleicht diese zumindest teilweise aus. Für das Schiebeelement 18 können die in Verbindung mit der Ausgleichsmasse 11 erläuterten Ausgestaltungen, insbesondere bezüglich der Massen und der Lagen der Massenschwerpunkte, entsprechend vorgesehen sein.

In der Fig. 3a ist die Vorrichtung zum Aufstechen eines organischen Gewebes in einer Betriebsstellung gezeigt, in welcher der Zapfen 7 und das zweite Antriebselement 8 in der jeweiligen distalen Position und der weitere Zapfen 17 und das Schiebeelement 18 in der jeweiligen proximalen Position angeordnet sind. Die Fig. 3b zeigt die Vorrichtung zum Aufstechen eines organischen Gewebes in einer Betriebsstellung, in welcher die Motorwelle 5 gegenüber der Betriebsstellung gemäß der Fig. 3a um eine halbe Umdrehung rotiert ist. Der Zapfen 7 und das zweite Antriebselement 8 sind in der jeweiligen proximalen Position angeordnet und der weitere Zapfen 17 und das Schiebeelement 18 sind in der jeweiligen distalen Position angeordnet.

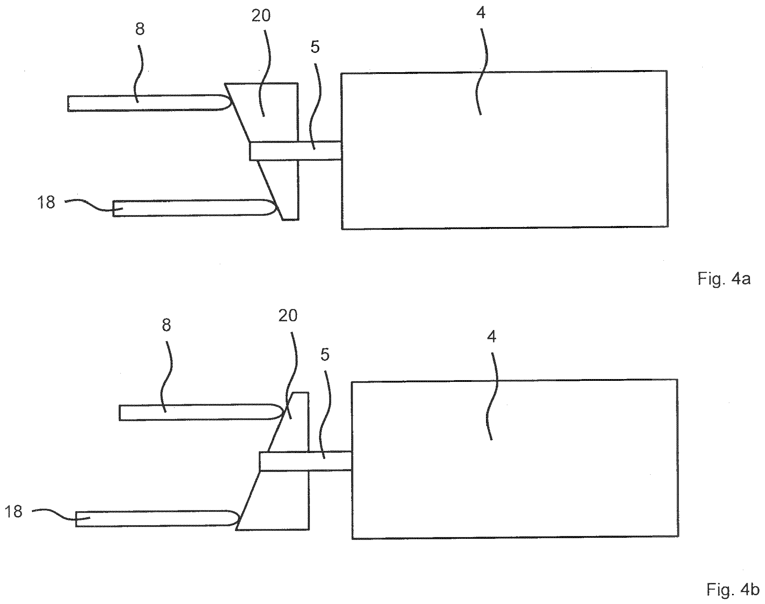

Die Fig. 4a und 4b zeigen eine Antriebsvorrichtung für eine Vorrichtung zum Aufstechen eines organischen Gewebes. Auf der Motorwelle 5 eines Motors 4 ist ein als eine Kurvenscheibe 20 bereitgestelltes erstes Antriebselement angeordnet. Die Kurvenscheibe 20 weist an einem distalen Ende (in den Figuren 4a und 4b links) ein Profil auf, welches entlang des Umfangs der Kurvenscheibe 20 in axialer Richtung veränderlich ist. In der Ausgestaltung gemäß den Figuren 4a und 4b ist das Profil durch eine Abschrägung des distalen Endes der Kurvenscheibe 20 bereitgestellt. Wie für Kurvenscheiben bekannt, kann das Profil der Kurvenscheibe 20 in Ausgestaltungen an einen gewünschten Bewegungsablauf angepasst sein.

Ein jeweiliges proximales Ende eines zweiten Antriebselements 8 und eines Schiebeelements 18 liegen an dem distalen Ende der Kurvenscheibe auf dem Profil auf. Bei einer Drehbewegung der Motorwelle 5 werden dann die proximalen Enden des zweiten Antriebselements 8 und des Schiebeelements 18 durch den Profilverlauf der Kurvenscheibe 20 vor und zurück bewegt. Die proximalen Enden des zweiten Antriebselements 8 und des Schiebeelements 18 können mit Hilfe geeigneter Rückhaltemittel (nicht dargestellt) gegen das Profil der Kurvenscheibe 20 gedrückt werden. Hierzu kann beispielsweise eine mechanische Feder genutzt werden. Alternativ können die proximalen Enden des zweiten Antriebselements 8 und des Schiebeelements 18 mit der Kurvenscheibe 20 verbunden werden, derart, dass diese bei einer Drehbewegung der Motorwelle 5 durch das Profil gedrückt und gezogen werden. Beispielsweise kann ein jeweiliges hervorstehendes Element des zweiten Antriebselements 8 und des Schiebeelements 18 in einen Profilhinterschnitt des Profils der Kurvenscheibe 20 greifen.

In der Fig. 4a ist die Antriebsvorrichtung in einer Betriebsstellung gezeigt, in welcher das zweite Antriebselement 8 in einer distalen Position und das Schiebeelement 18 in einer proximalen Position angeordnet sind. Die Fig. 4b zeigt die Antriebsvorrichtung in einer Betriebsstellung, in welcher die Motorwelle 5 gegenüber der Betriebsstellung gemäß der Fig. 4a um eine halbe Umdrehung rotiert ist. Das zweite Antriebselement 8 ist in einer proximalen Position angeordnet und das Schiebeelement 18 ist in einer distalen Position angeordnet.

In alternativen Ausgestaltungen kann die Kurvenscheibe ein Profil entlang ihres Umfangs aufweisen, wobei das Profil entlang des Umfangs der Kurvenscheibe 20 in axialer Richtung veränderlich ist. Beispielsweise kann das Profil mit einer Nut in der Mantelfläche der Kurvenscheibe gebildet sein, welche entlang des Umfangs der Kurvenscheibe in axialer Richtung einen Kurvenverlauf beschreibt. Das zweite Antriebselement 8 und das Schiebeelement 18 können zum Beispiel jeweils ein vorstehendes Element aufweisen, welches in dem Profil der Kurvenscheibe angeordnet ist, derart, dass der jeweilige Eingriffspunkt der vorstehenden Elemente des zweiten Antriebselements 8 und des Schiebeelements 18 bei einer Drehbewegung der Motorwelle 5 eine oszillierende Bewegung zwischen einer jeweiligen proximalen Position und einer jeweiligen distalen Position ausführen, welche jeweils auf das zweite Antriebselement 8 und das Schiebeelement 18 eingekoppelt werden.

In weiteren alternativen Ausgestaltungen kann die Kurvenscheibe ein Profil entlang ihres Umfangs aufweisen, wobei das Profil entlang des Umfangs der Kurvenscheibe 20 in radialer Richtung veränderlich ist. Es kann so der Radius der Kurvenscheibe, zumindest für einen Abschnitt der Kurvenscheibe, entlang des Umfangs veränderlich sein. Hierbei kann der Motor 4 mit der auf der Motorwelle 5 angeordneten Kurvenscheibe so angeordnet sein, dass sich ein Winkel zwischen der Längsachse des Motors 4 und der Längsachse des Gehäuses 1 um das zweite Antriebselement 8 und den Stößel 10, beispielsweise ein rechter Winkel, ergibt. Ein jeweiliges proximales Ende eines zweiten Antriebselements 8 können auf der Mantelfläche der Kurvenscheibe auf dem Profil aufliegen. Bei einer Drehbewegung der Motorwelle 5 wird dann das proximale Ende des zweiten Antriebselements 8 durch den Profilverlauf der Kurvenscheibe vor und zurück bewegt. Das proximale Ende des zweiten Antriebselements 8 kann mit Hilfe eines geeigneten Rückhaltemittels gegen das Profi! der Kurvenscheibe gedrückt werden. Hierzu kann beispielsweise eine mechanische Feder genutzt werden. Alternativ kann das proximale Ende des zweiten Antriebselements 8 mit der Kurvenscheibe verbunden werden, derart, dass dieses bei einer Drehbewegung der Motorwelle 5 durch das Profil gedrückt und gezogen wird. Beispielsweise kann ein hervorstehendes Element des zweiten Antriebselements 8 in einen Profilhinterschnitt des Profils der Kurvenscheibe greifen.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Vorrichtung zum Aufstechen eines organischen Gewebes, insbesondere einer menschlichen oder tierischen Haut, mit:
- einem Gehäuse (1);
- einer Stecheinrichtung (2), die in dem Gehäuse (1) an einem distalen Ende aufgenommen ist; und
- einer Antriebseinrichtung, die in dem Gehäuse (1) gebildet und proximal der Stecheinrichtung (2) angeordnet ist, mit:
- einem Antrieb (4), der eingerichtet ist, im Betrieb eine kontinuierliche Antriebsrotation bereitzustellen;
- einem ersten Antriebselement (6; 20), welches distal des Antriebs (4) angeordnet ist und funktionell an den Antrieb (4) koppelt, derart, dass die kontinuierliche Antriebsrotation des Antriebs (4) auf das erste Antriebselement (6; 20) eingekoppelt wird und zumindest ein Abschnitt des ersten Antriebselements (6; 20) im Betrieb eine kontinuierliche Rotationsbewegung ausführt;
- einem zweiten Antriebselement (8), welches im Wesentlichen distal des ersten Antriebselements (6; 20) angeordnet ist und über einen ersten Kopplungspunkt (7) an das erste Antriebselement (6; 20) funktionell koppelt, wobei
- die kontinuierliche Rotationsbewegung des zumindest einen Abschnitts des ersten Antriebselements (6; 20) im Betrieb eine oszillierende Bewegung des ersten Kopplungspunkts (7) zwischen einer proximalen Position und einer distalen Position des ersten Kopplungspunkts (7) bewirkt,
- die oszillierende Bewegung des ersten Kopplungspunkts (7) auf das zweite Antriebselement (8) eingekoppelt wird, derart, dass das zweite Antriebselement (8) eine oszillierende Antriebsbewegung ausführt, und
- das zweite Antriebselement (8) an die Stecheinrichtung (2) funktionell koppelt, derart, dass die oszillierende Antriebsbewegung des zweiten Antriebselements (8) auf die Stecheinrichtung (2) eingekoppelt wird; und
- einem Gegenelement (11; 18), welches distal des Antriebs (4) angeordnet ist und über einen zweiten Kopplungspunkt (13) an das erste Antriebselement (6; 20) funktionell koppelt, wobei
- die kontinuierliche Rotationsbewegung des zumindest einen Abschnitts des ersten Antriebselements (6; 20) im Betrieb eine oszillierende Bewegung des zweiten Kopplungspunkts (13) zwischen einer proximalen Position und einer distalen Position des zweiten Kopplungspunkts (13) bewirkt, und
- die oszillierende Bewegung des zweiten Kopplungspunkts (13) auf das Gegenelement (11; 18) eingekoppelt wird, derart, dass das Gegenelement (11; 18) eine oszillierende Elementbewegung ausführt;
wobei die oszillierende Bewegung des ersten Kopplungspunkts (7) und die oszillierende Antriebsbewegung des zweiten Antriebselements (8) gegenläufig zu der oszillierenden Bewegung des zweiten Kopplungspunkts (13) und der oszillierenden Elementbewegung des Gegenelements (11; 18) sind, so dass in dem Gehäuse (1) durch die oszillierende Antriebsbewegung des zweiten Antriebselements (8) verursachte Schwingungen wenigstens teilweise durch die oszillierende Elementbewegung des Gegenelements (11; 18) kompensiert werden.

2. Vorrichtung nach Anspruch 1, wobei eine Rotationsachse des zumindest einen Abschnitts des ersten Antriebselements (6; 20) im Wesentlichen parallel zu einer Längsachse des Gehäuses verläuft.

3. Vorrichtung nach Anspruch 1 oder 2, wobei das erste Antriebselement (6) eine Taumelscheibe umfasst.

4. Vorrichtung nach Anspruch 1 oder 2, wobei das erste Antriebselement (20) eine Kurvenscheibe umfasst.

5. Vorrichtung nach mindestens einem der vorhergehenden Ansprüche, wobei das Gegenelement (11) seitlich des ersten Antriebselements angeordnet und starr mit dem ersten Antriebselement verbunden ist.

6. Vorrichtung nach Anspruch 5, wobei die oszillierende Elementbewegung des Gegenelements (11) eine im Wesentlichen kreisbogenförmige oszillierende Bewegung ist.

7. Vorrichtung nach mindestens einem der Ansprüche 1 bis 4, wobei das Gegenelement (18) im Wesentlichen distal des ersten Antriebselements (6; 20) angeordnet und im Wesentlichen wie das zweite Antriebselement (8) geformt ist.

8. Vorrichtung nach Anspruch 7, wobei das Gegenelement (18) mit einem Gegenelement-Rückstellelement gekoppelt ist, welches auf das Gegenelement (18) eine Kraft ausübt, deren Richtung einer Antriebsrichtung des ersten Antriebselements (6; 20) entgegengesetzt ist.

9. Vorrichtung nach mindestens einem der vorhergehenden Ansprüche, wobei eine Masse des Gegenelements (11;18) im Wesentlichen gleich einer Masse des zweiten Antriebselements (8) ist.

10. Vorrichtung nach mindestens einem der vorhergehenden Ansprüche, wobei der Antrieb (4) ein Elektromotor ist.

11. Vorrichtung nach mindestens einem der vorhergehenden Ansprüche, wobei in dem Gehäuse (1) zumindest entlang einer Bewegungsrichtung der Stecheinrichtung (2) durch die oszillierende Antriebsbewegung des zweiten Antriebselements (8) verursachte Schwingungen im Wesentlichen vollständig durch die oszillierende Elementbewegung des Gegenelements (11; 18) kompensiert werden.

12. Antriebsmodul für eine Vorrichtung zum Aufstechen eines organischen Gewebes, insbesondere einer menschlichen oder tierischen Haut, mit:
- einem Modulgehäuse und
- einer Antriebseinrichtung, die in dem Modulgehäuse gebildet und konfiguriert ist, an einem distalen Ende des Modulgehäuses an eine Stecheinrichtung (2) eines Nadelmoduls zu koppeln, mit:
- einem Antrieb (4), der eingerichtet ist, im Betrieb eine kontinuierliche Antriebsrotation bereitzustellen;
- einem ersten Antriebselement (6; 20), welches distal des Antriebs (4) angeordnet ist und funktionell an den Antrieb (4) koppelt, derart, dass die kontinuierliche Antriebsrotation des Antriebs (4) auf das erste Antriebselement (6; 20) eingekoppelt wird und zumindest ein Abschnitt des ersten Antriebselements (6; 20) im Betrieb eine kontinuierliche Rotationsbewegung ausführt;
- einem zweiten Antriebselement (8), welches im Wesentlichen distal des ersten Antriebselements (6; 20) angeordnet ist und über einen ersten Kopplungspunkt (7) an das erste Antriebselement (6; 20) funktionell koppelt, wobei
- die kontinuierliche Rotationsbewegung des zumindest einen Abschnitts des ersten Antriebselements (6; 20) im Betrieb eine oszillierende Bewegung des ersten Kopplungspunkts (7) zwischen einer proximalen Position und einer distalen Position des ersten Kopplungspunkts (7) bewirkt,
- die oszillierende Bewegung des ersten Kopplungspunkts (7) auf das zweite Antriebselement (8) eingekoppelt wird, derart, dass das zweite Antriebselement (8) eine oszillierende Antriebsbewegung ausführt, und
- das zweite Antriebselement (8) eingerichtet ist, funktionell an die Stecheinrichtung (2) zu koppeln, derart, dass die oszillierende Antriebsbewegung des zweiten Antriebselements (8) auf die Stecheinrichtung eingekoppelt wird; und
- einem Gegenelement (11; 18), welches distal des Antriebs (4) angeordnet ist und über einen zweiten Kopplungspunkt (13) an das erste Antriebselement (6; 20) funktionell koppelt, wobei
- die kontinuierliche Rotationsbewegung des zumindest einen Abschnitts des ersten Antriebselements (6; 20) im Betrieb eine oszillierende Bewegung des zweiten Kopplungspunkts (13) zwischen einer proximalen Position und einer distalen Position des zweiten Kopplungspunkts (13) bewirkt, und
- die oszillierende Bewegung des zweiten Kopplungspunkts (13) auf das Gegenelement (11; 18) eingekoppelt wird, derart, dass das Gegenelement (11;18) eine oszillierende Elementbewegung ausführt;
wobei die oszillierende Bewegung des ersten Kopplungspunkts (7) und die oszillierende Antriebsbewegung des zweiten Antriebselements (8) gegenläufig zu der oszillierenden Bewegung des zweiten Kopplungspunkts (13) und der oszillierenden Elementbewegung des Gegenelements (11; 18) sind, so dass in dem Modulgehäuse durch die oszillierende Antriebsbewegung des zweiten Antriebselements (8) verursachte Schwingungen wenigstens teilweise durch die oszillierende Elementbewegung des Gegenelements (11; 18) kompensiert werden.
